# EUROPÄISCHE PATENTSCHRIFT

(11) **EP 0 824 515 B1**
(45) Veröffentlichungstag und Bekanntmachung des Hinweises auf die Patenterteilung: **04.04.2001**
(21) Anmeldenummer: 96912018.7
(22) Anmeldetag: 18.04.1996
(51) Int. Cl.: C07C 257/18, C07D 211/74, C07D 211/06, C07D 295/15, A61K 31/155, A61K 31/445, A61K 31/495

(54) **SUBSTITUIERTE PHENYLAMIDINE, DIESE VERBINDUNGEN ENTHALTENDE ARZNEIMITTEL UND VERFAHREN ZU IHRER HERSTELLUNG**
SUBSTITUTED PHENYL AMIDINES, MEDICAMENTS CONTAINING THESE COMPOUNDS AND PROCESS FOR PRODUCING THEM
PHENYLAMIDINES SUBSTITUEES, MEDICAMENTS CONTENANT CES COMPOSES ET LEURS PROCEDES DE PREPARATION

(30) Priorität: 27.04.1995 DE 19515500
(43) Veröffentlichungstag der Anmeldung: 25.02.1998
(73) Patentinhaber: Boehringer Ingelheim Pharma KG, 55218 Ingelheim am Rhein (DE)
(72) Erfinder: HIMMELSBACH, Frank, 88441 Mittelbiberach (DE); AUSTEL, Volkhard, 88400 Biberach (DE); LINZ, Günter, 88441 Mittelbiberach (DE); PIEPER, Helmut, 88400 Biberach (DE); GUTH, Brian, 88447 Warthausen (DE); WEISENBERGER, Johannes, 88400 Biberach (DE)
(74) Vertreter: Laudien, Dieter, Dr.
(86) Internationale Anmeldenummer: EP9601615
(87) Internationale Veröffentlichungsnummer: WO9633970

(56) Entgegenhaltungen:
- EP-A- 0 381 033
- EP-A- 0 483 667
- EP-A- 0 528 369
- EP-A- 0 539 343

## Beschreibung

In der Literatur werden bereits Fibrinogen-Rezeptor-Antagonisten beschrieben.

So fallen die Verbindungen der vorliegenden Erfindung unter den Schutzumfang der EP-A-0,483,667, werden aber in dieser nicht explicite beschrieben. Außerdem unterscheiden sich diese von denen der EP-A-0,381,033 durch die nicht vorhandene 1,4-Phenylengruppe, da der Rest B dem Rest Y in der vorliegenden Anmeldung entspricht, und von denen der EP-A-0,528,369 durch den Rest X₄, da X₄ keine -NH-CO-X-Gruppe darstellen kann, sowie von denen der EP-A-0,539,343 durch deren zweite -NH-CO-Gruppe.

Es wurde nun gefunden, daß die Phenylamidine der allgemeinen Formel 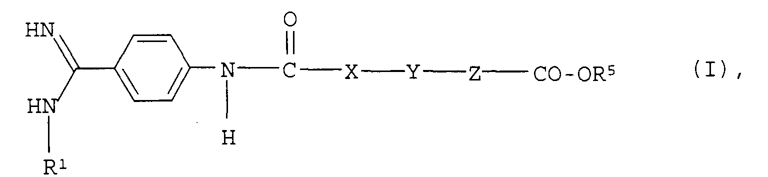 deren Tautomere, deren Stereoisomere einschließlich deren Gemische und deren Salze, insbesondere deren physiologisch verträgliche Salze mit anorganischen oder organischen Säuren oder Basen, ebenfalls wertvolle pharmakologische Eigenschaften aufweisen, vorzugsweise aggregationshemmende Wirkungen.

Gegenstand der vorliegenden Erfindung sind die obigen Verbindungen der allgemeinen Formel I, diese Verbindungen enthaltende Arzneimittel, deren Verwendung und Verfahren zu ihrer Herstellung.

In der obigen allgemeinen Formel I bedeutet
X eine Ethylengruppe, die durch eine oder zwei Methylgruppen substituiert sein kann,
Z eine Methylengruppe, die durch eine oder zwei Methylgruppen, durch eine Pyridyl- oder Phenylgruppe substituiert sein kann, wobei die Phenylgruppe zusätzlich durch ein Fluor-, Chloroder Bromatom, durch eine Methyl-, Methoxy- oder Trifluormethylgruppe substituiert sein kann,
Y eine 1,4-Cyclohexylen-, 1,4-Piperidinylen-, 2-Oxo-1,4-piperidinylen-, 1,4-Piperazinylen-, 2-Oxo-1,4-piperazinylen-, 2,3-Dioxo-1,4-piperazinylen- oder 2,5-Dioxo-1,4-piperazinylengruppe,
R¹ ein Wasserstoffatom, eine Alkyloxycarbonylgruppe mit insgesamt 2 oder 3 Kohlenstoffatomen oder eine Benzyloxycarbonylgruppe und
R⁵ ein Wasserstoffatom, eine Alkylgruppe mit 1 bis 4 Kohlenstoffatomen oder eine Cyclohexylgruppe.

Bevorzugte Verbindungen der obigen allgemeinen Formel I sind jedoch diejenigen, in denen
X eine Ethylengruppe,
Z eine Methylengruppe,
Y eine 1,4-Piperidinylengruppe,
R¹ ein Wasserstoffatom und
R⁵ ein Wasserstoffatom, eine Alkylgruppe mit 1 oder 2 Kohlenstoffatomen oder eine Cyclohexylgruppe bedeuten,
   deren Tautomere, deren Stereoisomere einschließlich deren Gemische und deren Salze.

Beispielsweise seien folgende besonders wertvolle Verbindungen der allgemeinen Formel I erwähnt:
4-[2-[(4-Amidinophenyl)aminocarbonyl]ethyl]-1-carboxymethylpiperidin,
4-[2-[(4-Amidinophenyl)aminocarbonyl]ethyl]-1-[(ethoxycarbonyl)-methyl]-piperidin und
4-[2-[(4-Amidinophenyl)aminocarbonyl]ethyl]-1-[(cyclohexyloxycarbonyl)methyl]-piperidin,
insbsondere jedoch 4-[2-[(4-Amidinophenyl) aminocarbonyl]-ethyl]-1-carboxymethyl-piperidin und dessen Salze.

Erfindungsgemäß erhält man die neuen Verbindungen der allgemeinen Formel I beispielsweise nach folgendem Verfahren:
Umsetzung einer Verbindung der allgemeinen Formel 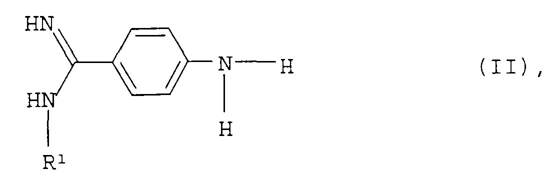 in der
R¹ wie eingangs definiert ist, mit einer Verbindung der allgemeinen Formel 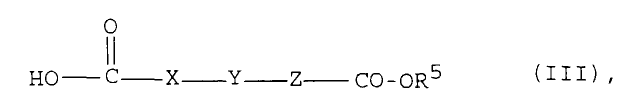 in der
R⁵, X, Y und Z wie eingangs definiert sind, oder deren reaktionsfähigen Derivaten und gegebenenfalls anschließende Überführung des Restes R⁵ in ein Wasserstoffatom.

Als reaktionsfähige Derivate einer Verbindung der allgemeinen Formel III kommen beispielsweise deren Säurechloride, Säureazide, gemischte Anhydride mit aliphatischen oder aromatischen Carbonsäuren oder Kohlensäuremonoester, deren Imidazolide und deren Ester wie deren Alkyl-, Aryl- und Aralkylester wie der Methyl-, Ethyl-, Isopropyl-, Pentyl-, Phenyl-, Nitrophenyloder Benzylester in Betracht.

Die Umsetzung wird zweckmäßigerweise in einem Lösungsmittel oder Lösungsmittelgemisch wie Methylenchlorid, Dimethylformamid, Dimethylsulfoxid, Benzol, Toluol, Chlorbenzol, Tetrahydrofuran, Pyridin, Pyridin/Dimethylformamid, Benzol/Tetrahydrofuran oder Dioxan gegebenenfalls in Gegenwart eines wasserentziehenden Mittels, z.B. in Gegenwart von Chlorameisensäureisobutylester, Thionylchlorid, Trimethylchlorsilan, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid, 2- (1H-Benztriazolyl)-1,1,3,3-tetramethyl-uronium-Salzen, N,N'-Carbonyldiimidazol, N,N'-Thionyldiimidazol oder Triphenylphosphin/Tetrachlorkohlenstoff, gegebenenfalls in Gegenwart von Dimethylaminopyridin oder 1-Hydroxy-benztriazol und/oder einer Base wie Triethylamin, N-Ethyl-diisopropylamin, Pyridin oder N-Methyl-morpholin, zweckmäßigerweise bei Temperaturen zwischen -10 und 180°C, vorzugsweise bei Temperaturen zwischen 0 und 120°C, durchgeführt.

Die anschließende Überführung des Restes R⁵ in ein Wasserstoffatom wird zweckmäßigerweise entweder in Gegenwart einer Säure wie Salzsäure, Schwefelsäure, Phosphorsäure, Essigsäure, Trichloressigsäure, Trifluoressigsäure oder deren Gemische oder in Gegenwart einer Base wie Lithiumhydroxid, Natriumhydroxid oder Kaliumhydroxid in einem geeigneten Lösungsmittel wie Wasser, Wasser/Methanol, Wasser/Ethanol, Wasser/Isopropanol, Methanol, Ethanol, Wasser/Tetrahydrofuran oder Wasser/Dioxan bei Temperaturen zwischen -10 und 120°C, z.B. bei Temperaturen zwischen Raumtemperatur und der Siedetemperatur des Reaktionsgemisches, durchgeführt.

Bei den vorstehend beschriebenen Umsetzungen können gegebenenfalls vorhandene reaktive Gruppen wie Carboxy-, Amino-, Alkylamino-, Imino- oder Amidinogruppen während der Umsetzung durch übliche Schutzgruppen geschützt werden, welche nach der Umsetzung wieder abgespalten werden.

Beispielsweise kommt als Schutzrest für eine Carboxylgruppe die Trimethylsilyl-, Methyl-, Ethyl-, tert.Butyl-, Benzyloder Tetrahydropyranylgruppe,
als Schutzrest für eine gegebenenfalls durch eine Alkylgruppe substituierte Amidinogruppe die Benzyloxycarbonylgruppe und
als Schutzrest für eine Amino-, Alkylamino- oder Iminogruppe die Formyl-, Acetyl-, Trifluoracetyl-, Ethoxycarbonyl-, tert.Butoxycarbonyl-, Benzyloxycarbonyl-, Benzyl-, Methoxybenzyl- oder 2,4-Dimethoxybenzylgruppe, für die Iminogruppe zusätzlich die Methylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder mittels Etherspaltung, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Temperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolg jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Ether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure oder Methanol bei Temperaturen zwischen 50 und 120°C oder durch Behandlung mit Natronlauge gegebenenfalls in Gegenwart eines Lösungsmittels wie Tetrahydrofuran oder Methanol bei Temperaturen zwischen 0 und 50°C.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Ferner können die erhaltenen Verbindungen der allgemeinen Formel I, wie bereits eingangs erwähnt wurde, gegebenenfalls in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel I mit mindestes 2 asymmetrischen Kohlenstoffatomen auf Grund ihrer physikalischchemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+) - oder (-)-Menthol und als optisch aktiver Acylrest in Amiden beispielsweise (+) -oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxylgruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Arginin, Cyclohexylamin, Ethanolamin, Dieethanolamin und Triethanolamin in Betracht.

Die als Ausgangsstoffe verwendeten Verbindungen sind teilweise literaturbekannt oder man erhält diese nach literaturbekannten Verfahren (siehe Beispiele).

Wie bereits eingangs erwähnt, weisen die neuen Phenylamidine der allgemeinen Formel I und deren Salze, insbesondere deren physiologisch verträgliche Salze mit anorganischen oder organischen Säuren oder Basen, wertvolle Eigenschaften auf. So weisen die neuen Verbindungen der allgemeinen Formel I wertvolle pharmakologische Eigenschaften auf, neben einer entzündungshemmenden und den Knochenabbau hemmenden Wirkung insbesondere antithrombotische, antiaggregatorische und tumor- bzw. metastasenhemmende Wirkungen.

Beispielsweise wurden die Verbindungen der allgemeinen Formel I auf ihre biologischen Wirkungen wie folgt untersucht:

### 1. Hemmung der Bindung von ³H-BIBU 52 an Humanthrombozyten:

Eine Suspension von Humanthrombozyten in Plasma wird mit ³H-BIBU 52 [= (3S,5S)-5-[(4'-Amidino-4-biphenylyl)oxymethyl]-3 - [(carboxy) methyl] -2-pyrrolidinon [3-3H-4-biphenylyl]], das den literaturbekannten Liganden ¹²⁵J-Fibrinogen ersetzt, (siehe DE-A-4,214.245) und verschiedenen Konzentrationen der zu testenden Substanz inkubiert. Der freie und gebundene Ligand wird durch Zentrifugation getrennt und durch Szintillationszählung quantitativ bestimmt. Aus den Meßwerten wird die Hemmung der ³H-BIBU 52-Bindung durch die Testsubstanz bestimmt.

Hierzu wird aus einer Antikubitalvene Spenderblut entnommen und mit Trinatriumzitrat antikoaguliert (Endkonzentration 13 mM). Das Blut wird 10 Minuten bei 170 x g zentrifugiert und das überstehende plättchenreiche Plasma (PRP) abgenommen. Das Restblut wird zur Gewinnung von Plasma nocheinmal scharf abzentrifugiert. Das PRP wird mit autologem Plasma 1:10 verdünnt. 750 µl werden mit 50 *µ*l physiologischer Kochsalzlösung, 100 *µ*l Testsubstanzlösung, 50 *µ*l ¹⁴C-Sucrose (3.700 Bq) und 50 *µ*l ³H-BIBU 52 (Endkonzentration: 5 nM) bei Raumtemperatur 20 Minuten inkubiert. Zur Messung der unspezifischen Bindung wird anstelle der Testsubstanz 5 *µ*l BIBU 52 (Endkonzentration: 30 *µ*M) eingesetzt. Die Proben werden 20 Sekunden bei 10000 x g zentrifugiert und der Überstand abgezogen. 100 *µ*l hiervon werden zur Bestimmung des freien Liganden gemessen. Das Pellet wird in 500 µl 0,2N NaOH gelöst, 450 µl werden mit 2 ml Szintillator und 25 *µ*l 5N HCl versetzt und gemessen. Das im Pellet noch verbliebene Restplasma wird aus dem ¹⁴C-Gehalt bestimmt, der gebundene Ligand aus der ³H-Messung. Nach Abzug der unspezifischen Bindung wird die Pelletaktivität gegen die Konzentration der Testsubstanz aufgetragen und die Konzentration für eine 50%ige Hemmung der Bindung ermittelt.

### 2. Antithrombotische Wirkung:

### Methodik

Die Thrombozytenaggregation wird nach der Methode von Born und Cross (J. Physiol. 170, 397 (1964)) in plättchenreichem Plasma gesunder Versuchspersonen gemessen. Zur Gerinnungshemmung wird das Blut mit Natriumcitrat 3,14 % im Volumenverhältnis 1:10 versetzt.

### Collagen-induzierte Aggregation

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München.

Vor der Collagen-Zugabe wird das Plasma jeweils 10 Minuten mit der Substanz bei 37°C inkubiert.

Aus den erhaltenen Meßzahlen wird graphisch eine EC₅₀ bestimmt, die sich auf eine 50%ige Änderung der "optical density" im Sinne einer Aggregationshemmung bezieht.

Die nachfolgende Tabelle enthält die gefundenen Ergebnisse:

| Substanz (Beispiel Nr.) | ³H-BIBU-52-Bindungstest IC₅₀[nM] | Hemmung der Plättchenaggregation EC₅₀ [nM] |
|---|---|---|
| 1 | 3,7 | 37 |
| 1(1) | 51 | 310 |

Auf Grund ihrer Hemmwirkung auf Zell-Zell- bzw. Zell-Matrix-Wechselwirkungen eignen sich die neuen cyclischen Harnstoffderivate der allgemeinen Formel I und ihre physiologisch verträglichen Salze zur Bekämpfung bzw. Verhütung von Krankheiten, bei denen kleinere oder größere Zell-Aggregate auftreten oder Zell-Matrixinteraktionen eine Rolle spielen, z.B. bei der Bekämpfung bzw. Verhütung von venösen und arteriellen Thrombosen, von zerebrovasculären Erkrankungen, von Lungenembolien, des Herzinfarktes, der Arteriosklerose, der Osteoporose und der Metastasierung von Tumoren und der Therapie genetisch bedingter oder auch erworbener Störungen der Interaktionen von Zellen untereinander oder mit soliden Strukturen. Weiterhin eignen sich diese zur Begleittherapie bei der Thrombolyse mit Fibrinolytica oder Gefäßinterventionen wie transluminaler Angioplastie oder auch bei der Therapie von Schockzuständen, der Psoriasis, des Diabetes und von Entzündungen.

Für die Bekämpfung bzw. Verhütung der vorstehend erwähnten Krankheiten liegt die Dosis zwischen 0,1 *µ*g und 30 mg/kg Körpergewicht, vorzugsweise bei 1 *µ*g bis 15 mg/kg Körpergewicht, bei bis zu 4 Gaben pro Tag. Hierzu lassen sich die erfindungsgemäß hergestellten Verbindungen der Formel I, gegebenenfalls in Kombination mit anderen Wirksubstanzen wie Thromboxan-Rezeptor-Antagonisten und Thromboxansynthesehemmer oder deren Kombinationen, Serotonin-Antagonisten, α-Rezeptorantagonisten, Alkylnitrate wie Glycerintrinitrat, Phosphodiesterasehemmer, Prostacyclin und deren Analoga, Fibrinolytica wie tPA, Prourokinase, Urokinase, Streptokinase, oder Antikoagulantien wie Heparin, Dermatansulfat, aktiviertes Protein C, Vitamin K-Antagonisten, Hirudin, Inhibitoren des Thrombins oder anderer aktivierter Gerinnungsfaktoren, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Stearylalkohol, Carboxymethylcellulose oder fetthaltigen Substanze wie Hartfett oder deren geeigneten Gemischen, in übliche galenische Zubereitungen wie Tabletten, Dragees, Kapseln, Pulver, Suspensionen, Lösungen, Sprays oder Zäpfchen einarbeiten.

Die nachfolgenden Beispiele sollen die Erfindung näher erläutern:

### Beispiel I

### 4-[2-(Chlorcarbonyl)ethyl]-1-[(ethoxycarbonyl)methyl]-piperidin-hydrochlorid

Zu 1,46 g 4- (2-Carboxyethyl) -1- [(ethoxycarbonyl)methyl] -piperidin in 10 ml Methylenchlorid wird unter Rühren 1 ml gesättigte etherische Salzsäure zugegeben. Es werden 1,2 g Thionylchlorid hinzugefügt und 3 Stunden bei Raumtemperatur gerührt. Das Reaktionsgemisch wird eingeengt und der Rückstand zweimal mit Toluol versetzt und jeweils wieder eingeengt. Das Rohprodukt wird ohne Reinigung in Beispiel 1 und 2 weiter umgesetzt.

Analog Beispiel I werden folgende Verbindungen erhalten:
(1) 1-[2-(Chlorcarbonyl)ethyl]-4-[(methoxycarbonyl)methyl]-piperidin-hydrochlorid
(2) 4-[2-(Chlorcarbonyl)ethyl]-1-[(cyclohexyloxycarbonyl)-methyl]-piperidin-hydrochlorid

### Beispiel II

### 4-[2-(Carboxy)ethyl]-1-[(ethoxycarbonyl)methyl]-piperidin

10 g 4-[2-(Benzyloxycarbonyl)ethyl]-1-[(ethoxycarbonyl)methyl]-piperidin werden in 150 ml Tetrahydrofuran 4 Stunden bei Raumtemperatur und einem Wasserstoffdruck von 50 psi in Gegenwart von 1,3 g Palladium auf Aktivkohle hydriert. Das Reaktionsgemisch eingeengt und mit Diethylether und wenig Aceton kristallisiert.
Ausbeute : 5,8 g (79 % der Theorie),
Schmelzpunkt : 65-67°C.

Analog Beispiel II wird folgende Verbindung erhalten:
(1) 4-(2-Carboxyethyl)-1-[(cyclohexyloxycarbonyl)methyl]-piperidin
Schmelzpunkt : 85-88°C.

### Beispiel III

### 4-[2-(Benzyloxycarbonyl)ethyl]-1-[(ethoxycarbonyl) methyl]-piperidin

Zu 9,0 g 4-[2-(Benzyloxycarbonyl)ethyl]-piperidin und 5,2 g N-Ethyl-diisopropylamin in 70 ml Acetonitril werden unter Rühren im Eisbad 6,35 g Bromessigsäureethylester in 20 ml Acetonitril zugetropft und 18 Stunden bei Raumtemperatur gerührt. Das Reaktionsgemisch wird eingeengt und der Rückstand rasch zwischen tert.Butyl-methylether, Eiswasser und 10 ml 2N Natronlauge verteilt. Die organische Phase wird abgetrennt mit Eiswasser und gesättigter Kochsalzlösung gewaschen, getrocknet und eingeengt.
Ausbeute : 10,05 g (83 % der Theorie),
R_{f}-Wert : 0,84 (Kieselgel; Methylenchlorid/Methanol/konz. wässriges Ammoniak = 95:5:1).

Analog Beispiel III wird folgende Verbindung erhalten:
(1) 4-[2-(Benzyloxycarbonyl)ethyl]-1-[(cyclohexyloxycarbonyl)-methyl] -piperidin
R_{f}-Wert : 0,47 (Kieselgel; Methylenchlorid/Methanol/konz. wäßriges Ammoniak = 98:2:0,5).

### Beispiel IV

### 4-[2-(Benzyloxycarbonyl)ethyl]-piperidin

9,7 g 4-(2-Carboxyethyl)piperidin-hydrochlorid (Schmelzpunkt:
240-250°C, hergestellt durch Hydrierung von 3-(4-Pyridyl)-acrylsäure in Eisessig in Gegenwart von Platinoxid und anschließender Behandlung mit Salzsäure), 30 ml Benzylalkohol, 3 g p-Toluolsulfonsäure und 50 ml Toluol werden 75 Minuten am Wasserabscheider erhitzt. Das Reaktionsgemisch wird im Vakuum eingeengt, der Rückstand mit 50 ml Eiswasser versetzt und dreimal mit tert.Butyl-methylether extrahiert. Die wäßrige Phase wird alkalisch gestellt und mit tert.Butyl-methylether extrahiert. Der Extrakt wird mit Kochsalzlösung gewaschen, getrocknet und eingeengt.
Ausbeute : 9,0 g (73 % der Theorie),
R_{f}-Wert : 0,18 (Kieselgel; Methylenchlorid/Methanol/konz. wässriges Ammoniak = 95:5:1).

### Beispiel V

### 1-(2-Carboxyethyl)-4-[(methoxycarbonyl)methyl]-piperidin-hydrochlorid

Zu 2,9 g 1-[2-(tert.Butoxycarbonyl)ethyl]-4-[(methoxycarbonyl)-methyl]-piperidin in 20 ml Methylenchlorid werden 10 ml Trifluoressigsäure gegeben und über Nacht bei Raumtemperatur gerührt. Das Reaktionsgemisch wird eingeengt, in Aceton aufgenommen, mit etherischer Salzsäure versetzt und erneut eingeengt. Es wird nochmals in Aceton aufgenommen, mit etherischer Salzsäure versetzt und eingeengt. Der Rückstand wird mit tert.Butyl-methylether, dem etwas Aceton zugesetzt wurde, gerührt, abgesaugt und getrocknet.
Ausbeute : 2,45 g (92 % der Theorie),
R_{f}-Wert : 0,73 (Reversed Phase Kieselgel; Methanol/5%ige wäßrig Kochsalzlösung = 6:4).

### Beispiel VI

### 1-[2-(tert.Butoxycarbonyl)ethyl]-4-[(methoxycarbonyl)methyl]-piperidin

Ein Gemisch aus 9 ml Acrylsäure-tert.butylester, 10 g 4-[(Methoxycarbonyl)methyl]-piperidin-hydrochlorid und 7,2 ml Triethylamin in 150 ml Methanol wird über Nacht unter Rückfluß erhitzt. Das Reaktionsgemich wird eingeengt, in Methylenchlorid aufgenommen und zweimal mit gesättigter Natriumhydrogencarbonat-Lösung gewaschen. Die organische Phase wird abgetrennt, eingeengt und der Rückstand durch Chromatographie über eine Kieselgelsäule mit Methylenchlorid/Methanol (35:5) gereinigt.
Ausbeute: 12,6 g (86 % der Theorie),
R_{f}-Wert: 0,68 (Kieselgel; Methylenchlorid/Methanol = 9:1).

### Beispiel 1

### 4-[2-[(4-Amidinophenyl)aminocarbonyl]ethyl]-1-carboxymethyl-piperidin x 0,2 H₂O

Zu 420 mg 4-Aminobenzamidin-dihydrochlorid und 20 mg 4-Dimethylaminopyridin in einem Gemisch aus 1,5 ml Dimethylformamid und 1,5 ml Pyridin werden 720 mg 4-[2-(Chlorcarbonyl)ethyl]-1- [(ethoxycarbonyl)methyl] -piperidin-hydrochlorid zugegeben und 1,3 Stunden bei 100°C gerührt. Das Reaktionsgemisch wird abgekühlt, mit Eiswasser versetzt, mit Natronlauge alkalisch gestellt und mit tert.Butyl-methylether und Methylenchlorid extrahiert. Die wässrige Phase wird mit Salzsäure auf einen pH-Wert von 3-4 gebracht und bei 70°C Badtemperatur zur Trockne eingedampft. Der Rückstand wird mit 100 ml Ethanol zum Sieden erhitzt, nach dem Abkühlen wird filtriert und das Filtrat eingeengt. Der Eindampfrückstand wird mit 30 ml Ethanol erhitzt, es wird abgekühlt und der Feststoff wird abgesaugt. Der Feststoff wird mit 15 ml Tetrahydrofuran und 4,5 ml 1N Natronlauge gerührt. Das Gemisch wird mit 2,75 ml 1N Salzsäure versetzt und im Eisbad gerührt. Der Niederschlag wird mit Wasser und Tetrahydrofuran gewaschen und im Vakuum getrocknet.
Ausbeute : 144 mg (21 % der Theorie),
Schmelzpunkt : 283°C (Zers.).
R_{f}-Wert : 0,76 (Reversed Phase Kieselgel; Methanol/5%ige wäßrige Kochsalzlösung = 6:4)

| | | | | | | |
|---|---|---|---|---|---|---|
| Ber. | C | 60,77 | H | 7,32 | N | 16,67 |
| Gef. | | 60,55 | | 7,26 | | 16,83 |

Massenspektrum : (M+H)⁺ = 333.

Analog Beispiel 1 werden folgende Verbindungen erhalten:
(1) 1-[2-[(4-Amidinophenyl)aminocarbonyl]ethyl]-4-carboxymethyl-piperidin x 2 H₂O
Schmelzpunkt: 192-200°C (unter Sintern und Zersetzung).
R_{f}-Wert : 0,77 (Reversed Phase Kieselgel; Methanol/5%ige wäßrige Kochsalzlösung = 6:4)

| | | | | | | |
|---|---|---|---|---|---|---|
| Ber. | C | 55,42 | H | 7,66 | N | 15,21 |
| Gef. | | 55,02 | | 7,38 | | 14,95 |

(2) 4-[2-[(4-Amidinophenyl)aminocarbonyl]ethyl]-1-carboxymethyl-piperazin
(3) 4-[2-[(4-Amidinophenyl)aminocarbonyl]ethyl]-1-carboxymethyl-2-oxo-piperidin
(4) 4-[2-[(4-Amidinophenyl)aminocarbonyl]ethyl]-1-carboxymethyl-2-oxo-piperazin
(5) 4-[2-[(4-Amidinophenyl)aminocarbonyl]ethyl]-1-carboxymethyl-3-methyl-2-oxo-piperazin
(6) 4-[2-[(4-Amidinophenyl)aminocarbonyl]ethyl]-1-carboxymethyl-2,3-dioxo-piperazin
(7) 4-[2-[(4-Amidinophenyl)aminocarbonyl]ethyl]-1-carboxymethyl-2,5-dioxo-piperazin
(8) 4-[2-[(4-Amidinophenyl)aminocarbonyl]ethyl]-1-carboxymethyl-3-oxo-piperazin
(9) 4-[2-[(4-Amidinophenyl)aminocarbonyl]ethyl]-1-carboxymethyl-cyclohexan
(10) 4-[2-[(4-Amidinophenyl)aminocarbonyl]ethyl]-1-(1-carboxyethyl)-piperidin
(11) α-[4-[2-[(4-Amidinophenyl)aminocarbonyl]ethyl]-1-piperidinyl]-phenylessigsäure
(12) α-[4-[2-[(4-Amidinophenyl)aminocarbonyl]ethyl]-1-piperidinyl]-(3-pyridyl)essigsäure
(13) 4-[2-[(4-Amidinophenyl)aminocarbonyl]propyl]-1-carboxymethyl-piperidin
(14) 4-[2-[(4-Amidinophenyl)aminocarbonyl]ethyl]-1-carboxymethyl-3,3-dimethyl-2-oxo-piperazin
(15) α-[4-[2-[(4-Amidinophenyl)aminocarbonyl]ethyl]-1-piperidinyl]-(4-fluorphenyl)essigsäure
(16) α-[4-[2-[(4-Amidinophenyl)aminocarbonyl]ethyl]-1-piperidinyl]-(4-methoxyphenyl)essigsäure
(17) 4-[2-[(4-Amidinophenyl)aminocarbonyl]ethyl]-1-(2-carboxyethyl)-piperidin

### Beispiel 2

### 4-[2-[(4-Amidinophenyl)aminocarbonyl]ethyl]-1-[(ethoxycarbonyl)methyl]-piperidin x 2,15 HCl x 0,7 H₂O

Zu 625 mg 4-Aminobenzamidin und 30 mg 4-Dimethylaminopyridin in 5 ml Pyridin werden 950 mg 4-[2-(Chlorcarbonyl)ethyl]-1-[(ethoxycarbonyl)methyl]-piperidin-hydrochlorid zugegeben und 1 Stunde bei 100°C gerührt. Es werden 2 ml Dimethylformamid zugegeben und weitere 1,2 Stunden bei 100°C gerührt. Das Reaktionsgemisch wird eingeengt und der Rückstand zweimal mit tert.Butyl-methylether gerührt, wobei jedesmal das Lösungsmittel abdekantiert und verworfen wird. Der Rückstand wird durch Chromatographie über Aluminiumoxid mit Ethanol gereinigt. Das Produkt wird in Ethanol gelöst, mit etherischer Salzsäure leicht sauer gestellt und eingeengt. Der Rückstand wird mit Aceton verrieben, der Festoff abgesaugt und getrocknet.
Ausbeute : 335 mg (25 % der Theorie),
R_{f}-Wert : 0,35 (Aluminiumoxid; Ethanol/konz. wäßriges Ammoniak = 99:1)

| | | | | | | | | |
|---|---|---|---|---|---|---|---|---|
| Ber. | C | 50,55 | H | 7,04 | N | 12,41 | Cl | 16,88 |
| Gef. | | 50,02 | | 6,96 | | 12,51 | | 17,27 |

Massenspektrum : (M+H)⁺ = 361.

Analog Beispiel 2 werden die Hydrochloride der folgenden Verbindungen erhalten:
(1) 1-[2-[(4-Amidinophenyl)aminocarbonyl]ethyl]-4-[(ethoxycarbonyl)methyl]-piperidin
(2) 4-[2-[(4-Amidinophenyl)aminocarbonyl]ethyl]-1-[(ethoxycarbonyl)methyl]-piperazin
(3) 4-[2-[(4-Amidinophenyl)aminocarbonyl]ethyl]-1-[(ethoxycarbonyl)methyl]-2-oxo-piperidin
(4) 4 [2-[(4-Amidinophenyl)aminocarbonyl]ethyl]-1-[(ethoxycarbonyl)methyl]-2-oxo-piperazin
(5) 4-[2-[(4-Amidinophenyl)aminocarbonyl]ethyl]-1-[(ethoxycarbonyl)methyl]-3-methyl-2-oxo-piperazin
(6) 4-[2-[(4-Amidinophenyl) aminocarbonyl]ethyl]-1-[(ethoxycarbonyl)methyl]-2,3-dioxo-piperazin
(7) 4-[2-[(4-Amidinophenyl)aminocarbonyl]ethyl]-1-[(ethoxycarbonyl)methyl]-2,5-dioxo-piperazin
(8) 4-[2-[(4-Amidinophenyl)aminocarbonyl]ethyl]-1-[(ethoxycarbonyl)methyl]-3-oxo-piperazin
(9) 4-[2-[(4-Amidinophenyl)aminocarbonyl]ethyl]-1-[(ethoxycarbonyl)methyl]-cyclohexan
(10) 4-[2-[(4-Amidinophenyl)aminocarbonyl]ethyl]-1-[1-(ethoxycarbonyl)ethyl]-piperidin
(11) α-[4-[2-[(4-Amidinophenyl)aminocarbonyl]ethyl]-1-piperidinyl]-phenylessigsäure-ethylester
(12) α-[4-[2-[(4-Amidinophenyl)aminocarbonyl]ethyl]-1-piperidinyl]-(3-pyridyl)essigsäure-ethylester
(13) 4-[2-[(4-Amidinophenyl)aminocarbonyl]propyl]-1-[(ethoxycarbonyl)methyl]-piperidin
(14) 4-[2-[(4-Amidinophenyl)aminocarbonyl]ethyl]-1-[(ethoxycarbonyl)methyl]-3,3-dimethyl-2-oxo-piperazin
(15) α-[4-[2- [(4-Amidinophenyl)aminocarbonyl] ethyl]-1-piperidinyl]-(4-fluorphenyl)essigsäure-ethylester
(16) α-[4-[2-[(4-Amidinophenyl)aminocarbonyl]ethyl]-1-piperidinyl]-(4-methoxyphenyl)essigsäure-ethylester
(17) 4-[2-[(4-Amidinophenyl)aminocarbonyl]ethyl]-1-[2-(ethoxycarbonyl)ethyl] -piperidin
(18) 4-[2-[(4-Amidinophenyl)aminocarbonyl]ethyl]-1-[(cyclohexyloxycarbonyl)methyl]-piperidin x 2,3 HCl x 2 H₂O
Schmelzpunkt : ab 170°C (Zers.).
R_{f}-Wert : 0,58 (Reversed Phase Kieselgel; Methanol/5%ige wäßrige Kochsalzlösung = 6:4)

| | | | | | | | | |
|---|---|---|---|---|---|---|---|---|
| Ber. | C | 51,69 | H | 7,60 | N | 10,48 | Cl | 15,26 |
| Gef. | | 51,69 | | 7,46 | | 10,44 | | 15,17 |

### Beispiel 3

| Trockenampulle mit 2,5 mg Wirkstoff pro 1 ml | |
|---|---|
| Zusammensetzung: | |
| Wirkstoff | 2,5 mg |
| Mannitol | 50,0 mg |
| Wasser für Injektionszwecke | ad 1,0 ml |

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel 4

| Trockenampulle mit 35 mg Wirkstoff pro 2 ml | |
|---|---|
| Zusammensetzung: | |
| Wirkstoff | 35,0 mg |
| Mannitol | 100,0 mg |
| Wasser für Injektionszwecke | ad 2,0 ml |

### Herstellung:

Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

### Beispiel 5

| Tablette mit 50 mg Wirkstoff | | |
|---|---|---|
| Zusammensetzung: | | |
| (1) | Wirkstoff | 50,0 mg |
| (2) | Milchzucker | 98,0 mg |
| (3) | Maisstärke | 50,0 mg |
| (4) | Polyvinylpyrrolidon | 15,0 mg |
| (5) | Magnesiumstearat | 2,0 mg |
| | | 215,0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

### Beispiel 6

| Tablette mit 350 mg Wirkstoff | | |
|---|---|---|
| Zusammensetzung: | | |
| (1) | Wirkstoff | 350,0 mg |
| (2) | Milchzucker | 136,0 mg |
| (3) | Maisstärke | 80,0 mg |
| (4) | Polyvinylpyrrolidon | 30,0 mg |
| (5) | Magnesiumstearat | 4,0 mg |
| | | 600,0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

### Beispiel 7

| Kapseln mit 50 mg Wirkstoff | | |
|---|---|---|
| Zusammensetzung: | | |
| (1) | Wirkstoff | 50,0 mg |
| (2) | Maisstärke getrocknet | 58,0 mg |
| (3) | Milchzucker pulverisiert | 50,0 mg |
| (4) | Magnesiumstearat | 2,0 mg |
| | | 160,0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

### Beispiel 8

| Kapseln mit 350 mg Wirkstoff | | |
|---|---|---|
| Zusammensetzung: | | |
| (1) | Wirkstoff | 350,0 mg |
| (2) | Maisstärke getrocknet | 46,0 mg |
| (3) | Milchzucker pulverisiert | 30,0 mg |
| (4) | Magnesiumstearat | 4,0 mg |
| | | 430,0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

## Patentansprüche

1. Phenylamidine der allgemeinen Formel 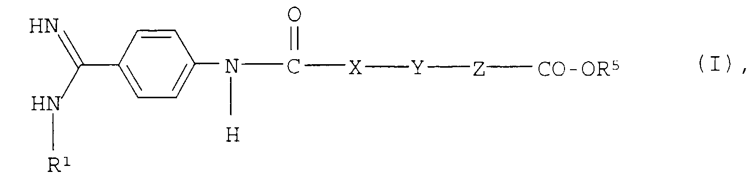 in der
X eine Ethylengruppe, die durch eine oder zwei Methylgruppen substituiert sein kann,
Z eine Methylengruppe, die durch eine oder zwei Methylgruppen, durch eine Pyridyl- oder Phenylgruppe substituiert sein kann, wobei die Phenylgruppe zusätzlich durch ein Fluor-, Chloroder Bromatom, durch eine Methyl-, Methoxy- oder Trifluormethylgruppe substituiert sein kann,
Y eine 1,4-Cyclohexylen-, 1,4-Piperidinylen-, 2-Oxo-1,4-piperidinylen-, 1,4-Piperazinylen-, 2-Oxo-1,4-piperazinylen-, 2,3-Dioxo-1,4-piperazinylen- oder 2,5-Dioxo-1,4-piperazinylengruppe,
R¹ ein Wasserstoffatom, eine Alkyloxycarbonylgruppe mit insgesamt 2 oder 3 Kohlenstoffatomen oder eine Benzyloxycarbonylgruppe und
R⁵ ein Wasserstoffatom, eine Alkylgruppe mit 1 bis 4 Kohlenstoffatomen oder eine Cyclohexylgruppe bedeuten,
deren Tautomere, deren Stereoisomere einschließlich deren Gemische und deren Salze.

2. Phenylamidine der allgemeinen Formel I gemäß Anspruch 1, in der
X eine Ethylengruppe,
Z eine Methylengruppe,
Y eine 1,4-Piperidinylengruppe,
R¹ ein Wasserstoffatom und
R⁵ ein Wasserstoffatom, eine Alkylgruppe mit 1 oder 2 Kohlenstoffatomen oder eine Cyclohexylgruppe bedeuten,
deren Tautomere, deren Stereoisomere einschließlich deren Gemische und deren Salze.

3. Folgende Verbindungen der allgemeinen Formel I gemäß Anspruch 1:
4-[2-[(4-Amidinophenyl)aminocarbonyl]ethyl]-1-carboxymethylpiperidin,
4-[2-[(4-Amidinophenyl)aminocarbonyl]ethyl]-1-[(ethoxycarbonyl)-methyl]-piperidin und
4-[2-[(4-Amidinophenyl)aminocarbonyl]ethyl]-1-[(cyclohexyloxycarbonyl)methyl]-piperidin
sowie deren Salze.

4. 4-[2-[(4-Amidinophenyl) aminocarbonyl]ethyl]-1-carboxymethyl-piperidin und dessen Salze.

5. Physiologisch verträgliche Salze der Verbindungen nach mindestens einem der Ansprüche 1 bis 4 mit anorganischen oder organischen Säuren oder Basen.

6. Arzneimittel, enthaltend eine Verbindung nach mindestens einem der Ansprüche 1 bis 4 oder ein physiologisch verträgliches Salz gemäß Anspruch 5 neben gegebenenfalls einem oder mehreren inerten Trägerstoffen und/oder Verdünnungsmitteln.

7. Verwendung einer Verbindung nach mindestens einem der Ansprüche 1 bis 5 zur Herstellung eines Arzneimittels, das zur Bekämpfung bzw. Verhütung von Krankheiten, bei denen kleinere oder größere Zell-Aggregate auftreten oder Zell-Matrixinteraktionen eine Rolle spielen, geeignet ist.

8. Verfahren zur Herstellung eines Arzneimittels gemäß Anspruch 6, dadurch gekennzeichnet, daß auf nichtchemischem Wege eine Verbindung nach mindestens einem der Ansprüche 1 bis 5 in einen oder mehrere inerte Trägerstoffe und/oder Verdünnungsmittel eingearbeitet wird.

9. Verfahren zur Herstellung der Verbindungen der allgemeinen Formel I gemäß den Ansprüchen 1 bis 5, dadurch gekennzeichnet, daß
eine Verbindung der allgemeinen Formel 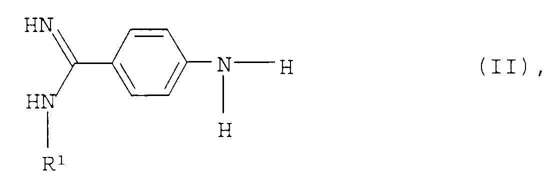 in der
R¹ wie in den Ansprüchen 1 bis 4 definiert ist, mit einer Verbindung der allgemeinen Formel 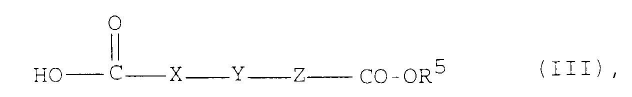 in der
R⁵, X, Y und Z wie in den Ansprüchen 1 bis 4 definiert sind, oder deren reaktionsfähigen Derivaten umgesetzt und
gegebenenfalls anschließend eine so erhaltene Verbindung der allgemeinen Formel I, in der R⁵ kein Wasserstoffatom darstellt, in eine Verbindung der allgemeinen Formel I, in der R5 ein Wasserstoffatom darstellt, übergeführt wird und/oder
erforderlichenfalls ein bei den vorstehend beschriebenen Umsetzungen verwendeter Schutzrest wieder abgespalten wird und/oder
gewünschtenfalls eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträgliche Salze übergeführt wird.

## Claims

1. Phenylamidines of general formula 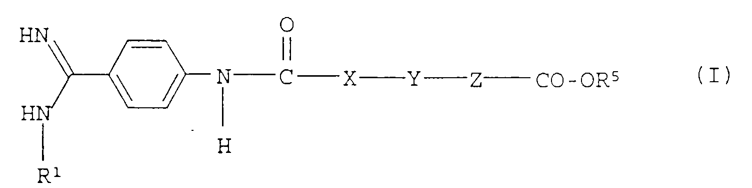 wherein
X denotes an ethylene group, which may be substituted by one or two methyl groups,
Z denotes a methylene group, which may be substituted by one or two methyl groups, by a pyridyl or phenyl group, wherein the phenyl group may additionally be substituted by a fluorine, chlorine or bromine atom or by a methyl, methoxy or trifluoromethyl group,
Y denotes a 1,4-cyclohexylene, 1,4-piperidinylene, 2-oxo-1,4-piperidinylene, 1,4-piperazinylene, 2-oxo-1,4-piperazinylene, 2,3-dioxo-1,4-piperazinylene or 2,5-dioxo-1,4-piperazinylene group,
R¹ denotes a hydrogen atom, an alkyloxycarbonyl group having a total of 2 or 3 carbon atoms or a benzyloxycarbonyl group, and
R⁵ denotes a hydrogen atom, an alkyl group having 1 to 4 carbon atoms or a cyclohexyl group,
the tautomers thereof, the stereoisomers including the mixtures thereof and the salts thereof.

2. Phenylamidines of general formula I according to claim 1, wherein
X denotes an ethylene group,
Z denotes a methylene group,
Y denotes a 1,4-piperidinylene group,
R¹ denotes a hydrogen atom and
R⁵ denotes a hydrogen atom, an alkyl group having 1 or 2 carbon atoms or a cyclohexyl group,
the tautomers thereof, the stereoisomers including the mixtures thereof and the salts thereof.

3. The following compounds of general formula I according to claim 1:
4-[2-[(4-amidinophenyl)aminocarbonyl]ethyl]-1-carboxymethyl-piperidine,
4-[2-[(4-amidinophenyl)aminocarbonyl]ethyl]-1-[(ethoxycarbonyl)-methyl]-piperidine and
4-[2-[(4-amidinophenyl)aminocarbonyl]ethyl]-1-[(cyclohexyloxycarbonyl)methyl]-piperidine,
and the salts thereof.

4. 4-[2-[(4-amidinophenyl)aminocarbonyl]ethyl]-1-carboxymethyl-piperidine, and the salts thereof.

5. Physiologically acceptable salts of the compounds according to at least one of claims 1 to 4 with inorganic or organic acids or bases.

6. Pharmaceutical compositions containing a compound according to at least one of claims 1 to 4 or a physiologically acceptable salt according to claim 5 optionally together with one or more inert carriers and/or diluents.

7. Use of a compound according to at least one of claims 1 to 5 for preparing a pharmaceutical composition which is suitable for combating or preventing diseases in which smaller or larger cell aggregations occur or cell-matrix interactions play a part.

8. Process for preparing a pharmaceutical composition according to claim 6, characterised in that a compound according to at least one of claims 1 to 5 is incorporated in one or more inert carriers and/or diluents by a non-chemical method.

9. Process for preparing the compounds of general formula I according to claims 1 to 5, characterised in that
a compound of general formula 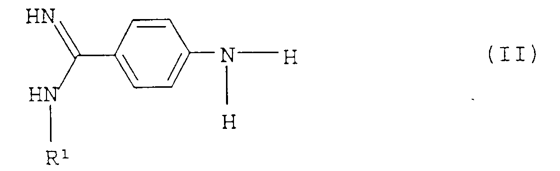 wherein
R¹ is defined as in claims 1 to 4,
is reacted with a compound of general formula 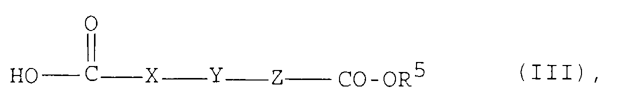 wherein
R⁵, X, Y and Z are defined as in claims 1 to 4, or the reactive derivatives thereof, and
subsequently, if desired, a compound of general formula I thus obtained wherein R⁵ does not denote a hydrogen atom is converted into a compound of general formula I wherein R⁵ denotes a hydrogen atom, and/or
if necessary a protective group used in the reactions described hereinbefore is cleaved again and/or
if desired, a compound of general formula I thus obtained is resolved into its stereoisomers and/or
a compound of general formula I thus obtained is converted into the salts thereof, particularly, for pharmaceutical use, into the physiologically acceptable salts thereof.

## Revendications

1. Phénylamidines de formule générale 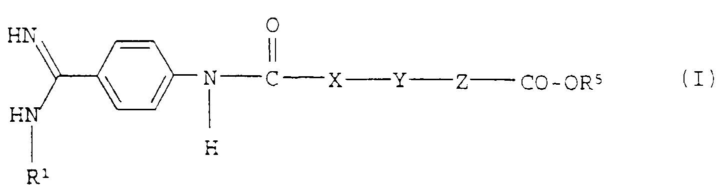 dans laquelle
X représente un groupe éthylène qui peut être substitué par un ou deux groupes méthyle,
Z représente un groupe méthylène qui peut être substitué par un ou deux groupes méthyle, par un groupe pyridyle ou phényle, le groupe phényle pouvant être substitué en outra par un atome de fluor, de chlore ou de brome, par un groupe méthyle, méthoxy ou trifluorométhyle,
Y représente un groupe 1,4-cyclohexylène, 1,4-pipéridinylène, 2-oxo-1,4-pipéridinylène, 1,4-pipérazinylène, 2-oxo-1,4-pipérazinylène, 2,3-dioxo-1,4-pipérazinylène ou 2,5-dioxo-1,4-pipérazinylène,
R¹ représente un atome d'hydrogène, un groupe alkyloxycarbonyle ayant au total 2 ou 3 atomes de carbone ou un groupe benzyloxycarbonyle et
R⁵ représente un atome d'hydrogène, un groupe alkyle ayant 1 à 4 atomes de carbone ou un groupe cyclohexyle,
leurs tautomères, leurs stéréoisoméres y compris leurs mélanges et leurs sels.

2. Phénylamidines de formule générale I selon la revendication 1 dans laquelle
X représente un groupe éthylène,
Z représente un groupe méthylène,
Y représente un groupe 1,4-pipéridinylène,
R¹ représente un atome d'hydrogène et
R⁵ représente un atome d'hydrogène, un groupe alkyle ayant 1 ou 2 atomes de carbone ou un groupe cyclohexyle,
leurs tautomères, leurs stéréoisomères y compris leurs mélanges et leurs sels.

3. Composés de formule générale I selon la revendication 1 suivants :
4- [2-[(4-amidinophényl)aminocarbonyl]éthyl]-1-carboxyméthyl-pipéridine,
4- [2-[(4-amidinophényl)aminocarbonyl]éthyl]-1-[(éthoxycarbonyl)méthyl]pipéridine et
4- [2-[(4-amidinophényl)aminocarbonyl]éthyl]-1-[(cyclohexyloxycarbonyl)méthyl]pipéridine
ainsi que leurs sels.

4. 4-[2-[(4-amidinophènyl)aminocarbonyl]éthyl]-1-carboxyméthylpipéridine et ses sels.

5. Sels physiologiquement acceptables des composés selon au moins l'une des revendications 1 à 4 avec des acides ou bases inorganiques ou organiques.

6. Médicament contenant un composé selon au moins l'une des revendications 1 à 4 ou un sel physiologiquement acceptable selon la revendication 5 et éventuellement un ou plusieurs supports et/ou diluants inertes.

7. Utilisation d'un composé selon au moins l'une des revendications 1 à 5 pour la préparation d'un médicament qui convient pour la lutte contre ou la prévention de maladies dans lesquelles des agrégats cellulaires de taille relativement petite ou relativement grande apparaissent ou les interactions cellule-matrice jouent un rôle.

8. Procédé de préparation d'un médicament selon la revendication 6 caractérisé en ce que, par voie non chimique, un composé selon au moins l'une des revendications 1 à 5 est incorporé dans un ou plusieurs supports et/ou diluants inertes.

9. Procédé de préparation des composés de formule générale I selon les revendications 1 à 5 caractérisé en ce que
un composé de formule générale 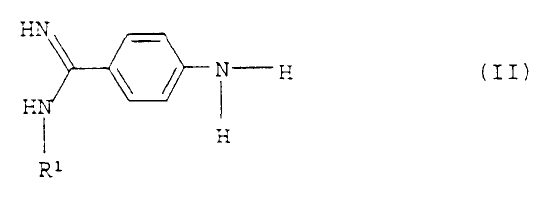 dans laquelle
R¹ est défini comme dans les revendications 1 à 4 est mis à réagir avec un composé de formule générale 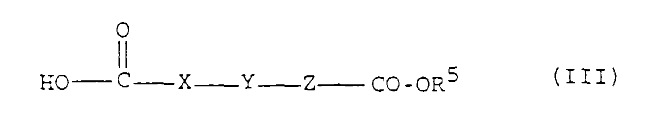 dans laquelle
R⁵, X, Y et Z sont définis comme dans les revendications 1 à 4 ou avec ses dérivés réactifs puis
éventuellement un composé ainsi obtenu, de formule générale I dans laquelle R⁵ ne représente pas un atome d'hydrogène, est converti en un composé de formule générale I dans laquelle R⁵ représente un atome d'hydrogène, et/ou
si nécessaire, un reste protecteur utilisé dans les réactions décrites précédemment est clivé et/ou
si on le souhaite, un composé de formule générale I ainsi obtenu est séparé en ses stéréoisomères et/ou
un composé de formule générale I ainsi obtenu est converti en ses sels, en particulier pour l'utilisation pharmaceutique en ses sels physiologiquement acceptables.
